# EUROPEAN PATENT SPECIFICATION

(11) **EP 2 653 130 B1**
(45) Date of publication and mention of the grant of the patent: **24.12.2014**
(21) Application number: 13164607.7
(22) Date of filing: 19.04.2013
(51) Int. Cl.: A61B 19/00

(54) **Support and guide device for a surgical instrument, in particular for neurosurgery**
Träger und Führungsvorrichtung für ein chirurgisches Instrument, insbesondere für Neurochirurgie
Dispositif de support et de guidage pour instrument chirurgical, en particulier pour la neurochirurgie

(30) Priority: 20.04.2012 IT MI20120663
(43) Date of publication of application: 23.10.2013
(73) Proprietor: Promev S.r.l., Lecco (IT)
(72) Inventor: Dallolio, Villiam, 23900 Lecco (IT)
(74) Representative: Boggio, Luigi

(56) References cited:
- WO-A1-02/13714
- WO-A1-98/17191
- WO-A1-98/51214
- US-A- 4 998 938
- US-A1- 2008 183 191

## Description

The present invention concerns a support and guide device for a surgical instrument, in particular for neurosurgery.

Various neurosurgical procedures (for example biopsy and cerebral endoscopy) are performed with instruments that reach the brain via a hole made in the skull and by means of guided imaging techniques, i.e. assisted by neuronavigator.

Different devices are known which support and guide the surgical instrument in this type of operation.

Examples of support and guide devices for neurosurgical instruments are shown in WO 02/13714 and WO 2008/013709.

US 2008/0183191 discloses a device with the features of the preamble of claim 1.

The known devices can be improved, however, in particular in terms of production simplicity and costs, ease of use, effectiveness and in particular with reference to the fastening system of the device to the skull.

One object of the present invention is to provide a support and guide device for a surgical instrument, in particular for neurosurgery, which is particularly simple and effective, specifically allowing simple, rapid and safe fastening to the skull.

The present invention therefore concerns a support and guide device for a surgical instrument, in particular for neurosurgery, as defined in essential terms in the attached Claim 1 and, in its additional characteristics, in the dependent claims.

The device of the invention is particularly simple and effective, specifically allowing simple, rapid and safe fastening to the cranial bone.

In particular, the device of the invention does not require the hole made in the bone to be threaded, thus permitting a less invasive procedure on the patient; the device ensures in any case a stable safe fastening, able to withstand also relatively heavy instruments (endoscopes with camera).

Furthermore, the device can be easily applied and accurately adjusted.

The invention is further described in the following nonlimiting embodiment examples, with reference to the attached figures in which:
- figure 1 is a schematic view in longitudinal section of a support and guide device for a surgical instrument in accordance with the invention, shown in use (i.e. fixed to a skull);
- figure 2 is an exploded perspective view of the device of figure 1;
- figures 3 and 4 are two longitudinal section views, according to respective orthogonal planes, of the device of figure 2, assembled;
- figures 5 and 6 are a lateral view and a longitudinal section view respectively of a detail of the device of the invention according to a different embodiment;
- figure 7 is a lateral view of another detail of the device of the invention, according to a variation;
- figures 8 and 9 are longitudinal section views of the detail of figure 7, in two alternative configurations.

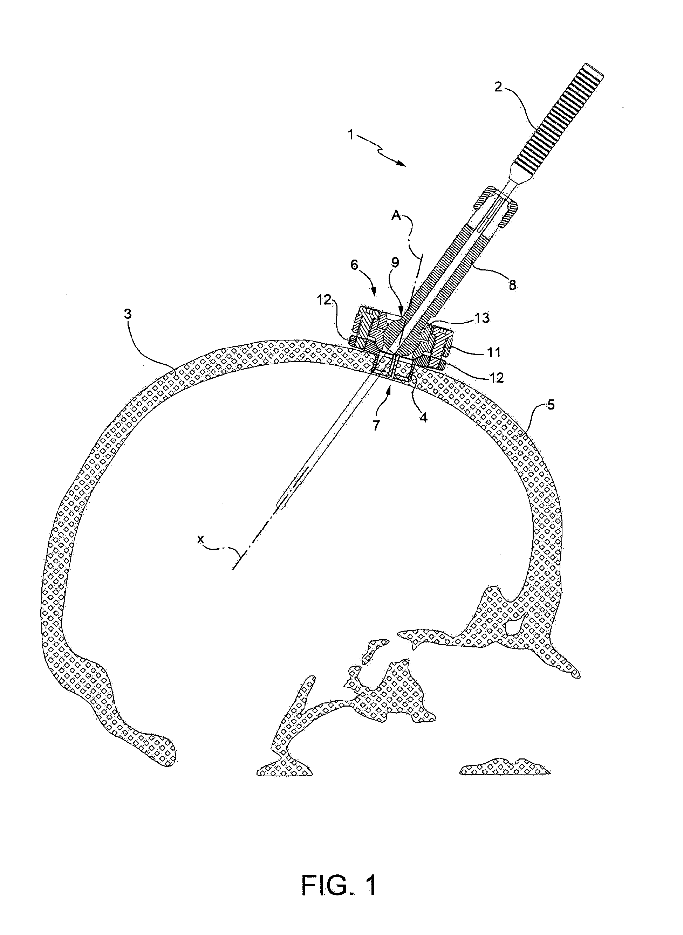

In figure 1, the number 1 indicates as a whole a support and guide device for a surgical instrument 2, in particular for neurosurgery.

The device 1 is attachable to the skull 3 of a patient, at a hole 4 made in the cranial bone 5, for supporting and guiding the instrument 2 which is, for example (but not only), a cerebral biopsy probe or forceps, an endoscope, an electrode, etc.

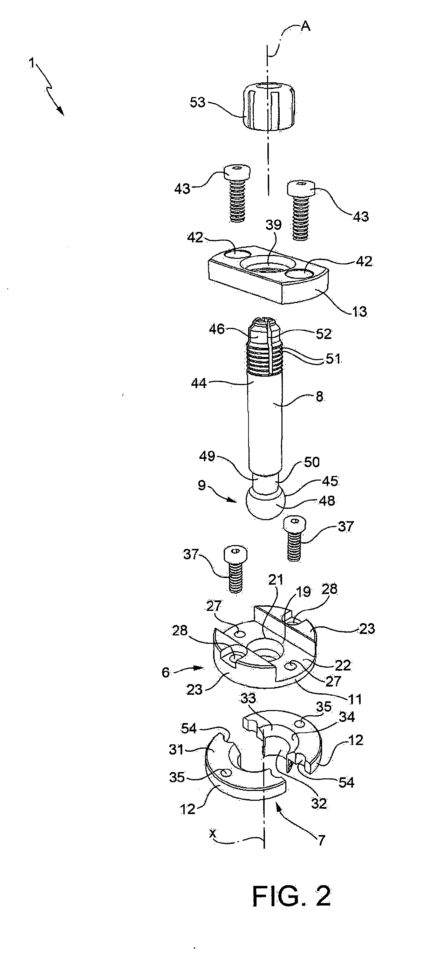

With reference also to figure 2, the device 1 comprises a base body 6, provided with an expansion fastening system 7 for fastening the device 1 to the skull 3, a guide rod 8 which receives in use the instrument 2, and a ball joint 9 which joins the guide rod 8 to the base body 6.

The base body 6 extends substantially along and about an axis A which coincides, in use, with a central axis of the hole 4; the base body 6 comprises a central element 11, two fastening elements 12 cooperating with the central element 11, and a locking plate 13.

In the example shown in figures 1-4, the elements 11, 12 and the plate 13 consist of respective separate pieces, for example made of metal material, of the base body 6.

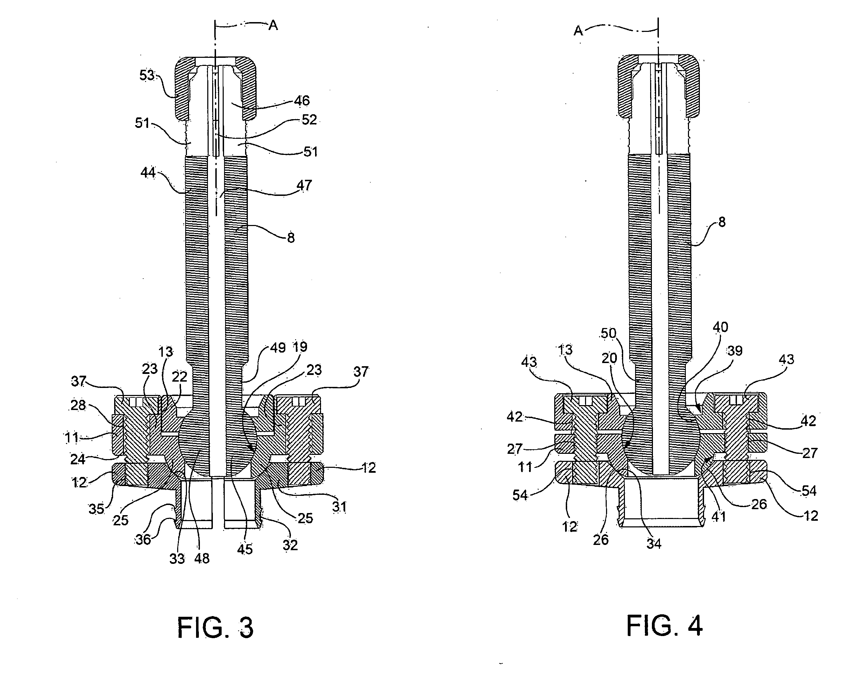

With reference also to figures 3 and 4, the central element 11 is substantially disc-shaped and has a central aperture 19 passing along the axis A; the aperture 19 has a curved inner lateral surface 20, in particular shaped as a portion of a spherical bowl.

An upper face 21 of the central element 11 is provided with a cavity 22, delimited by two lateral projections 23 and in which the plate 13 is housed. On a lower face 24, opposite to the upper face 21, of the element 11, two hinge portions 25 project (which can be parts of a continuous annular collar) facing each other on opposite sides of the axis A and having respective outer contact surfaces 26, preferably curved and/or rounded, convex to be more precise.

The element 11 is provided with two pairs of holes 27, 28 angularly offset by 90° from each other.

The fastening elements 12 are symmetrical with respect to the axis A and arranged diametrically opposite with respect to the axis A and facing each other; the elements 12 are radially spaced from each other, i.e. they are separated crosswise to the axis A. Each element 12 is crescent-shaped and has a flange portion 31, facing the lower face 24 of the central element 11, and a collar portion 32, which projects downwardly from the flange portion 31 about the axis A. The flange portion 31 is provided with a cavity 33 flared towards the axis A and having a contact surface 34 facing towards the element 11 and inclined with respect to the axis A; the flange portion 31 is also provided with a threaded hole 35, aligned with a hole 28 of the central element 11. The collar portion 32 has radially outer teeth 36, which project radially from an outer lateral surface of the collar portion 32. Preferably, the teeth 36 are spaced from one another both angularly (in a circumferential direction about the axis A) and axially (parallel to the axis A); the teeth 36 are for example arranged in circumferential rows (in particular, at least two rows) spaced axially (parallel to the axis A) from one another; each row is formed of several teeth 36 angularly spaced from one another.

The crescent-shaped fastening elements 12 are positioned below the lower face 24 of the central element 11; the hinge portions 25 of the element 11 are inserted in respective cavities 33 of the elements 12 and rest on the surfaces 34 of the cavities 33 with their respective surfaces 26.

Adjusting screws 37 are inserted through the holes 28 of the element 11 and engage the holes 35 of the elements 12. The elements 12 are joined to the element 11 by means of the adjusting screws 37, which also exert a return action on the elements 12, as will be clarified below.

The locking plate 13 is arranged in the cavity 22 and has a central aperture 39 passing along the axis A, aligned and contiguous with the aperture 19 of the element 11 and having a curved inner lateral surface 40, in particular shaped as a portion of a spherical bowl, opposite the surface 20; the two apertures 19, 39 with the respective surfaces 20, 40 define together a rotation seat 41 of the joint 9.

The plate 13 is provided with two diametrically opposite holes 42, positioned on opposite sides of the aperture 39 and aligned with respective holes 27 of the element 11. The plate 13 is joined to the element 11 by means of locking screws 43 arranged through respective holes 42 and which engage respective holes 27.

The guide rod 8, preferably made of polymeric material, for example Radel® polyphenylsulfone (PPSU), has a rectilinear stem 44 which extends along an axis X between two opposite ends 45, 46 and is provided with an inner longitudinal through channel 47 in which the instrument 2 is inserted in use. The end 45 has a bulb 48, which is substantially spherical and is inserted in the seat 41 and defines with it the ball joint 9; the bulb 48 is joined to the stem 44 by means of a restricted portion 49 having a diameter lower than the diameter of the bulb 48 and defined by a circumferential groove 50. The end 46 has a plurality of longitudinal sectors 51 angularly spaced apart from one another and separated by longitudinal slits 52 parallel to the axis X; the sectors 51 are externally threaded to receive a locking ring nut 53 which, screwed onto the sectors 51, tightens them towards one another and towards the axis X, locking the instrument 2 in the channel 47.

In use, the device 1 is fastened to the skull 3 (in which the hole 4 has been made) by means of the expansion fastening system 7, formed in particular by the adjusting screws 37, by the hinge portions 25 and by the fastening elements 12.

First the fastening elements 12 are mounted on the central element 11, by means of the adjusting screws 37 which are inserted through the holes 28 of the element 11 and engage the holes 35 of the elements 12; the adjusting screws 37 are left loose so that the fastening elements 12 can move with respect to the central element 11 and the collar portions 32 can be easily inserted in the hole 4.

The fastening elements 12 are connected to the central element 11 by means of the hinge portions 25 which allow the fastening elements 12 to flex with respect to the axis A and with respect to the central element 11; the adjusting screws 37 act on the fastening elements 12 to flex and then expand the fastening elements 12 and, to be more precise, to stretch the fastening elements 12 apart from each another.

When the adjusting screws 37 are tightened in the respective holes 35, the adjusting screws 37 pull the flange portions 31 of the elements 12 towards the element 11 and consequently flex the collar portions 32, due to the contact between the surfaces 26, 34. The fastening elements 12 diverge from each other, expanding inside the hole 4; the teeth 36 engage in the hole 4, fastening the device 1 to the skull 3.

The guide rod 8 is then inserted through the aperture 39 of the plate 13, with the bulb 48 on the side of the surface 40, and the plate 13 is positioned in the cavity 22 of the element 11 so that the bulb 48 is housed in the seat 41. The plate 13 is fixed to the element 11 by means of the locking screws 43 which are positioned through the holes 42 and engage the holes 27, terminating in the space between the fastening elements 12 (if necessary in seats 54 defined by pairs of opposite recesses formed on the fastening elements 12); the guide rod 8 is oriented with respect to the base body 6 by means of the ball joint 9, with the bulb 48 rotating in the seat 41; the conformation of the end 45, and in particular the presence of the groove 50, allow an inclination of the guide rod 8 with respect to the base body 6 of at least 20° per side. When the locking screws 43 are tightened, the guide rod 8 is locked in the required position.

The instrument 2 is then inserted through the channel 47 and locked by means of the ring nut 53 which tightens the sectors 51 around the instrument 2.

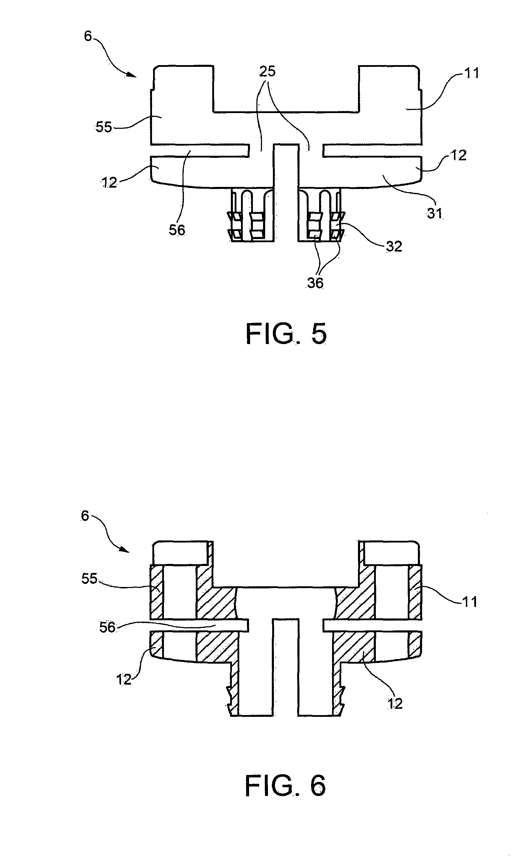

In the variation shown in figures 5 and 6, in which the details similar or identical to those already described are indicated by the same numbers, the central element 11 and the fastening elements 12 consist of respective portions of a monolithic body 55, preferably made of polymeric material, for example Radel® polyphenylsulfone (PPSU).

The element 11 comprises hinge portions 25, consisting in this case of opposite C-shaped portions of the monolithic body 55.

In all other respects, the device 1 is similar to what has been described previously; in particular, it is always the adjusting screws 37 (not illustrated in figures 5-6) that exert a return action on the fastening elements 12, flexing the elements 12 via the hinge portions 25.

The fastening elements 12 comprise respective flange portions 31, facing the lower face 24 of the central element 11, and respective collar portions 32, which project downwardly from the flange portions 31 about the axis A. The flange portions 32 are spaced axially from the lower face 24 of the element 11 and are separated from it by an interstice 56. The collar portions 32 have the radially outer teeth 36 already described; in particular, the teeth project radially from an outer lateral surface of the collar portion 32 and are spaced from one another both angularly and axially, since they are arranged on at least two circumferential rows axially spaced from each other; each row is formed of several teeth 36 spaced angularly from one another.

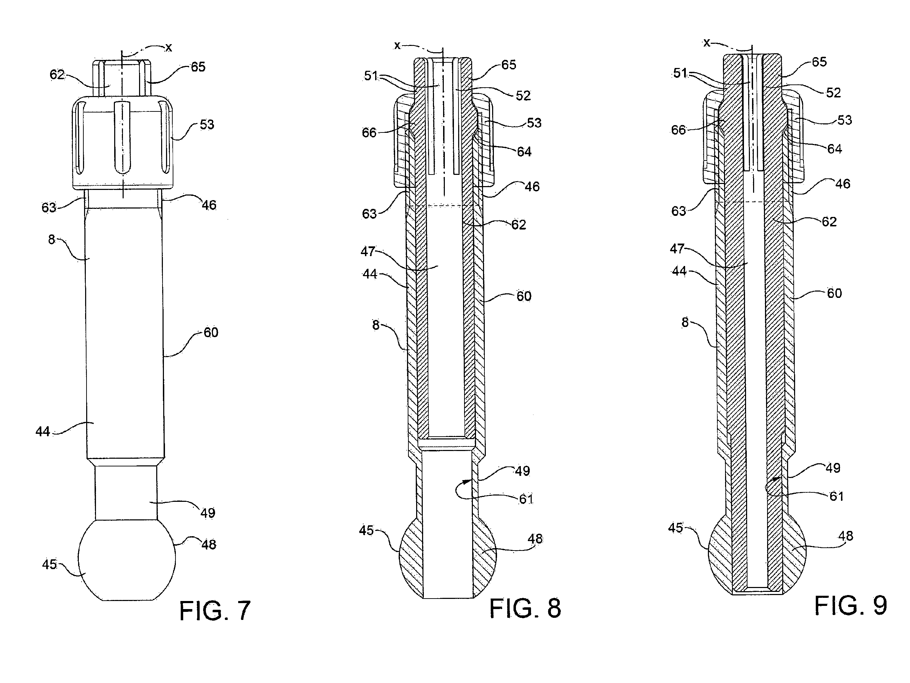

According to the variation illustrated in figures 7-9, the guide rod 8 comprises an outer tubular casing 60 (for example made of metal material) having an internal longitudinal through seat 61, and a series of tubular inserts 62 (preferably made of polymeric material such as Radel®) which are interchangeable and can be selectively inserted in the seat 61.

The casing 60 comprises the stem 44 and, at the end 45 of the stem 44, the bulb 48 and the restricted portion 49 described previously; the opposite end 46 has a threaded outer portion 63 to receive the locking ring nut 53 and terminates with an annular edge 64.

Each insert 62 is shaped like a substantially cylindrical tubular element and has an axial end 65 formed of a plurality of longitudinal sectors 51 angularly spaced from one another and separated by longitudinal slits 52 parallel to the axis X; the sectors 51 have respective radially outer protrusions 66 cooperating with and abutting against the edge 64. Each insert 62 has an internal longitudinal through channel 47 for the insertion of an instrument 2. The inserts 62 of the series all have the same outer diameter, so as to be housed in the seat 61, and different internal diameter (diameter of the channel 47) to house various instruments 2. The inserts 62 can also have a different length; two interchangeable inserts 62 are shown by way of example in figures 8 and 9.

In this way, according to the instrument 2 to be used, the insert 62 having internal diameter suitable to receive and support the instrument 2 can be selected. The selected insert 62 is inserted in the seat 61 until it abuts, with the protrusions 66, against the edge 64; when the ring nut 53 is tightened on the threaded portion 63 of the end 46, the ring nut 53 pulls the sectors 51 towards each other, locking the instrument 2 in the channel 47. This variation reduces any movement of the instrument 2 inserted in the guide rod 8; the instrument 2 is in fact locked both by the sectors 51 and the ring nut 53, and by the inner surface of the insert 62.

Lastly, it is understood that further modifications and variations that do not depart from the scope of the attached claims can be made to the device described and illustrated here.

## Claims

1. A support and guide device (1) for a surgical instrument, in particular for neurosurgery, comprising a base body (6), extending substantially along and about an axis (A) and provided with a fastening system (7) for fixing the device (1) to a bone, a guide rod (8) having a channel (47) for receiving in use a surgical instrument, and a ball joint (9) that joins the guide rod (8) with the base body (6), the fastening system (7) is an expansion fastening system, comprising fastening elements (12); the device (1) being **characterized in that** said fastening elements (12) are connected to a central element (11) via hinge portions (25) that allow the fastening elements (12) to flex with respect to the axis (A) and to the central element (11); and adjusting screws (37) that act on the fastening elements (12) for flexing and hence expanding the fastening elements (12).

2. A device according to Claim 1, wherein the fastening elements (12) are positioned diametrically opposite to each other with respect to the axis (A) and are separated from each other crosswise to the axis (A).

3. A device according to Claim 1 or 2, wherein the central element (11) and the fastening elements (12) consist of respective distinct pieces, in particular made of metal material, of the base body (6); and wherein the hinge portions (25) project from a bottom face (24) of the central element (11) and have respective first outer contact surfaces (26), curved and convex and cooperating with respective second contact surfaces (34) of the fastening (12) elements.

4. A device according to Claim 1 or 2, wherein the central element (11) and the fastening elements (12) consist of respective portions of a monolithic body (55), preferably made of a polymer material, and the hinge portions (25) consist of opposite C-shaped portions of the monolithic body (55).

5. A device according to one of the preceding claims, wherein each fastening element (12) is crescent-shaped and has a flange portion (31), facing the bottom face (24) of the central element (11), and a collar portion (32) that projects downwardly from the flange portion (31) about the axis (A) and is provided with radially outer teeth (36).

6. A device according to Claim 5, wherein the teeth (36) are spaced from one another both angularly and axially.

7. A device according to Claim 5 or 6, wherein the teeth (36) are arranged on at least two circumferential rows axially spaced from one another; each row being formed by a plurality of teeth (36) angularly spaced apart from one another.

8. A device according to one of the preceding claims, wherein the adjusting screws (37) are housed through respective first holes (28) formed in the central element (11) and engage respective second threaded holes (35) formed in the fastening elements (12).

9. A device according to one of the preceding claims, wherein the base body (6) comprises a locking plate (13) joined to the central element (11) via locking screws (43); the ball joint (9) comprising a rotation seat (41), defined between the locking plate (13) and the central element (11), and a bulb (48) positioned at an end (45) of the guide rod (8) and housed in the rotation seat (41).

10. A device according to Claim 9, wherein the bulb (48) is joined to a stem (44) of the guide rod (8) via a restricted portion (49) having a diameter lower than the diameter of the bulb (48) and defined by a circumferential groove (50).

## Patentansprüche

1. Eine Träger und Führungsvorrichtung (1) für ein chirurgisches Instrument, insbesondere zur Neurochirurgie. aufweisend einen Basiskörper (6), der sich im Wesentlichen entlang einer Achse (A) und um die Achse (A) erstreckt und mit einem Befestigungssystem (7) zum Befestigen der Vorrichtung (1) an einem Knochen versehen ist, eine Führungsstange (8), die einen Kanal (47) hat, um bei einer Verwendung ein chirurgisches Instrument aufzunehmen, und ein Kugelgelenk (9), welches die Führungsstange (8) mit dem Basiskörper (6) verbindet, wobei das Befestigungssystem (7) ein Expansionsbefestigungssystem ist. das Befestigungselemente (12) beinhaltet, wobei die Vorrichtung (1) **dadurch gekennzeichnet ist, dass** die Befestigungselemente (12) mit einem Zentralelement (11) über Gelenkabschnitte (25), welche es dem Befestigungselementen (12) gestatten, sich mit Bezug zu der Achse (A) und dem Zentralelement (11) zu beugen, und über Einstellungsschrauben (37), die auf die Befestigungselemente (12) zum Beugen und somit und somit zum Expandieren der Befestigungselemente (12) wirken, verbunden sind.

2. Eine Vorrichtung gemäß Anspruch 1, wobei die Befestigungselemente (12) mit Bezug zu der Achse (A) zueinander diametral gegenüber positioniert sind, und diese voneinander kreuzweise zu der Achse (A) beabstandet sind.

3. Eine Vorrichtung gemäß Anspruch 1 oder 2, wobei das Zentralelement (11) und die Befestigungselemente (12) aus jeweiligen verschiedenen Teilen, die insbesondere aus einem Metall-Material gemacht, des Basiskörpers (6) bestehen, und wobei die Gelenkabschnitte (25) von einer Grundseite (24) des Zentralelements (11) auskragen und jeweilige erste äußere Kontaktoberflächen (26) haben, die gekurvt und konvex sind, und die mit jeweiligen zweiten Kontaktoberflächen (34) der Befestigungselemente (12) zusammenwirken.

4. Eine Vorrichtung gemäß Anspruch 1 oder 2, wobei das Zentralelement (11) und die Befestigungselemente (12) aus jeweiligen Abschnitten eines monolithischen Körpers (55) bestehen, der bevorzugt aus einem Polymer-Material gemacht ist, und wobei die Gelenkabschnitte (25) aus gegenüberliegenden C-förmigen Abschnitten des monolithischen Körpers (55) bestehen.

5. Eine Vorrichtung gemäß einen der voranstehenden Ansprüchen, wobei jedes Befestigungselement (12) sichelförmig ist und einen Flankenabschnitt (31), der der Grundseite (24) des Zentralelements (11) zugewandt ist, und einen Kragenabschnitt (32) hat, der von dem Flankenabschnitt (31) um die Achse (A) nach unten ragt und mit der radialen Außenzähnen (36) versehen ist.

6. Eine Vorrichtung gemäß Anspruch 5, wobei die Zähne (36) von einander sowohl winkelbezogen als auch axial beabstandet sind.

7. Eine Vorrichtung gemäß Anspruch 5 oder 6, wobei die Zähne (36) an zumindest zwei Umfangsreihen, die voneinander axial beabstandet sind, angeordnet sind, wobei jede Reihe durch eine Vielzahl an Zähnen (36) gebildet ist, welche voneinander winkelbezogen beabstandet sind.

8. Eine Vorrichtung gemäß einem der voranstehenden Ansprüche, wobei die Einstellungsschrauben (37) durch jeweilige erste Löcher (28), die in dem Zentralelement (11) gebildet sind, aufgenommen werden und mit jeweiligen zweiten Gewindelöchern (35), die in den Befestigungselementen (12) gebildet sind, in Wirkverbindung stehen.

9. Eine Vorrichtung gemäß einem der voranstehenden Ansprüche, wobei der Basiskörper (6) eine Sperrplatte (13) beinhaltet, die mit dem Zentralelement (11) über Sperrschrauben (34) verbunden ist, wobei das Kugelgelenk (9) einen Rotationssitz (41), der zwischen der Sperrplatte (13) und dem Zentralelement (11) definiert ist, und ein kugelartiges Gebilde (48), das an einem Ende (45) der Führungsstange (8) positioniert ist, und das in dem Rotationssitz (41) aufgenommen ist, beinhaltet.

10. Eine Vorrichtung gemäß Anspruch 9, wobei das kugelartiges Gebilde (48) mit einem Stamm (44) der Führungsstange (8) über einen abgegrenzten Abschnitt (49) verbunden ist. der eine geringeren Durchmesser als den Durchmesser des kugelartigen Gebildes (48) hat, und der durch eine Umfangsnut (50) definiert ist.

## Revendications

1. Dispositif de support et de guidage (1) pour un instrument chirurgical, en particulier pour la neurochirurgie, comprenant un corps de base (6), s'étendant sensiblement le long et autour d'un axe (A) et doté d'un système de fixation (7) pour fixer le dispositif (1) à un os, un arbre de guidage (8) ayant un canal (47) pour recevoir durant l'utilisation un instrument chirurgical, et une articulation sphérique (9) qui joint l'arbre de guidage (8) au corps de base (6), le système de fixation (7) est un système de fixation par expansion, comprenant des éléments de fixation (12) ; le dispositif (1) étant **caractérisé en ce que** lesdits éléments de fixation (12) sont raccordés à un élément central (11) via des parties charnières (25) qui permettent que les éléments de fixation (12) fléchissent relativement à l'axe (A) et à l'élément central (11) ; et des vis d'ajustement (37) qui agissent sur les éléments de fixation (12) pour fléchir et par conséquent déployer les éléments de fixation (12).

2. Dispositif selon la revendication 1, dans lequel les éléments de fixation (12) sont positionnés diamétralement à l'opposé les uns des autres relativement à l'axe (A) et sont séparés les uns des autres transversalement à l'axe (A).

3. Dispositif selon la revendication 1 ou 2, dans lequel l'élément central (11) et les éléments de fixation (12) sont constitués de différentes pièces respectives, en particulier faites en un matériau métallique, du corps de base (6) ; et dans lequel les parties charnières (25) font saillie à partir d'une face inférieure (24) de l'élément central (11) et ont des premières surfaces de contact extérieures respectives (26), incurvées et convexes et coopérant avec des secondes surfaces de contact respectives (34) des éléments de fixation (12).

4. Dispositif selon la revendication 1 ou 2, dans lequel l'élément central (11) et les éléments de fixation (12) sont constitués de parties respectives d'un corps monolithique (55), de préférence faits en un matériau polymère, et les parties charnières (25) sont constituées de parties opposées en forme de C du corps monolithique (55).

5. Dispositif selon l'une des revendications précédentes, dans lequel chaque élément de fixation (12) a une forme de croissant et a une partie bride (31), face à la surface inférieure (24) de l'élément central (11), et une partie collerette (32) qui fait saillie vers le bas à partir de la partie bride (31) autour de l'axe (A) et est dotée de dents radialement extérieures (36).

6. Dispositif selon la revendication 5, dans lequel les dents (36) sont espacées les unes des autres angulairement et axialement.

7. Dispositif selon la revendication 5 ou 6, dans lequel les dents (36) sont agencées sur au moins deux rangées circonférentielles axialement espacées les unes des autres ; chaque rangée étant formée par une pluralité de dents (36) angulairement espacées les unes des autres.

8. Dispositif selon l'une des revendications précédentes, dans lequel les vis d'ajustement (37) sont logées dans des premiers trous respectifs (28) formés dans l'élément central (11) et se mettent en prise avec des seconds trous filetés respectifs (35) formés dans les éléments de fixation (12).

9. Dispositif selon l'une des revendications précédentes, dans lequel le corps de base (6) comprend une plaque de blocage (13) jointe à l'élément central (11) via des vis de blocage (43) ; l'articulation à balle (9) comprenant un siège de rotation (41), défini entre la plaque de blocage (13) et l'élément central (11), et un bulbe (48) positionné au niveau d'une extrémité (45) de l'arbre de guidage (8) et logé dans le siège de rotation (41).

10. Dispositif selon la revendication 9, dans lequel le bulbe (48) est joint à une tige (44) de l'arbre de guidage (8) via une partie restreinte (49) ayant un diamètre inférieur au diamètre du bulbe (48) et défini par un sillon circonférentiel (50).
